(19) **Europäisches Patentamt**
**European Patent Office**
**Office européen des brevets**

(11) **EP 1 764 104 B1**

(12) **EUROPEAN PATENT SPECIFICATION**

(45) Date of publication and mention
of the grant of the patent:
**13.08.2008 Bulletin 2008/33**

(51) Int Cl.:
*A61K 31/70* (2006.01)   *A61K 33/06* (2006.01)
*A61K 31/07* (2006.01)   *A61K 31/355* (2006.01)
*A61K 31/198* (2006.01)   *A61P 17/02* (2006.01)

(21) Application number: **05425649.0**

(22) Date of filing: **16.09.2005**

(54) **Wound healing pharmaceutical composition comprising sucralfate, glycine, aluminium acetate and vitamins**

Wundheilende pharmazeutische Zusammensetzung enthaltend Sucralfat, Glycin, Aluminium Acetat und Vitamine

Composition pharmaceutique à base de sucralfate, de glycine, d'acétate d'aluminium et de vitamines pour la guérison des plaies

(84) Designated Contracting States:
**AT BE BG CH CY CZ DE DK EE ES FI FR GB GR HU IE IS IT LI LT LU LV MC NL PL PT RO SE SI SK TR**

(43) Date of publication of application:
**21.03.2007 Bulletin 2007/12**

(73) Proprietor: **Italfar Società a Responsabilità Limitata**
**00040 Pomezia (RM) (IT)**

(72) Inventor: **Russo, Domenico**
**89100 Reggio (Calabria) (IT)**

(74) Representative: **Long, Giorgio et al**
**Jacobacci & Partners S.p.A.**
**Via Senato, 8**
**20121 Milano (IT)**

(56) References cited:
WO-A-89/03232    WO-A-89/05645
WO-A-92/09289    WO-A-02/062353
WO-A-02/089818    FR-A- 2 646 604
US-A- 4 373 519    US-A- 4 945 084
US-A1- 2004 157 766

- MARKHAM T ET AL: "Topical sucralfate for erosive irritant diaper dermatitis." ARCHIVES OF DERMATOLOGY. OCT 2000, vol. 136, no. 10, October 2000 (2000-10), pages 1199-1200, XP009061668 ISSN: 0003-987X
- GUNDERSEN R Y ET AL: "Glycine--an important neurotransmitter and cytoprotective agent." ACTA ANAESTHESIOLOGICA SCANDINAVICA. SEP 2005, vol. 49, no. 8, September 2005 (2005-09), pages 1108-1116, XP002367453 ISSN: 0001-5172

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention relates to a pharmaceutical composition having wound-healing activity.

Sucralfate is an amorphous complex of aluminium hydroxide and sucrose sulphate, identified by the name: β-D-fructofuranosyl-α-D-glucopyranoside octakis (hydrogen sulphate) aluminium complex.

Pharmaceutical compositions containing sucralfate as active ingredient are currently used as antiulcer treatments. Indeed, sucralfate has the ability to inhibit pepsin activity, to create a protective layer impeding the action of hydrochloric acid on the gastric mucosa, and to bind bile salts, neutralising their aggressive action in relation to the mucosa itself.

WO 89/05645 relates to wound-healing compositions comprising sucralfate, optionally containing vitamins and aluminium.

The present invention is aimed at sucralfate-containing compositions which have shown activities other than those known to date.

In a first aspect, the present invention relates to a composition comprising sucralfate, aluminium acetate, at least one vitamin selected from: vitamin A, C, or E, preferably vitamin A, and glycine.

More particularly, the composition of the invention also includes appropriate quantities of emollient and hydrating substances, such as for example lanolin and vaseline.

In a further aspect, the composition of the invention also contains an antimycotic agent, preferably selected from econazole, miconazole or itraconazole and/or an antibacterial agent selected from erythromycin, gentamycin, neomycin, colloidal silver or silver sulphadiazine.

The composition of the invention possesses cytoprotective, isolating and carrier activities. In particular, sucralfate possess all three such activities. Indeed, sucralfate has the capacity to bind to proteins at the base of epidermal wounds and sores forming, along with the exudate, a protective film which shelters the wound from external cytotoxic agents and ensures the prolonged persistence of other active substances within the ulceration sites.

Glycine is the simplest aminoacid and possesses cytoprotective action because it provides the biological basis for the revitalisation processes of metabolically stressed cells. Furthermore, it inhibits peripheral nerve fibre conductance with the consequent reduction of pain symptoms.

Vitamin A is essential for collagen formation and epidermal keratinisation. Its activities are also expressed in the differentiation of newly formed cells and intervening in their growth. Vitamin A also has anti-carcinogenic activity, particularly useful in cell differentiation processes.

Aluminium acetate is a compound with astringent and antiseptic activities. It is only absorbed by the cell membranes of the base of the wound, with no absorption with systemic effects.

Hence, the composition of the invention has epidermal wound healing activity and is particularly useful in healing and protecting epithelial ulcerations, both those that have been produced by external agents: burns, wounds of various origins etc., and by ischaemic phenomena: diabetic ulcers and bedsores. The composition is also used as a gynaecological wound-healing agent.

In the case where the composition also includes an antibiotic or antifungal agent, it may be used to treat bacterially and fungally infected wounds and sores respectively. In the case of the latter, the composition is also envisaged for gynaecological use.

The quantities of the various components used for the preparation of the composition of the invention are reported in the following table 1.

Table 1.

| Component | Quantity, expressed as % by weight | Preferred quantity, expressed as % by weight |
|---|---|---|
| Sucralfate | 1-6 | 2-5 |
| Aluminium acetate | 0.5-3.5 | 1-2.5 |
| Glycine | 1-6 | 2-5 |
| Vitamins | 0.2-3 | 0.8-1.5 |
| Antibacterial agents | 0.01-5 | 0.1-4 |
| Antifungal agents | 0.01-5 | 2-4 |
| Emollients, hydrating agents | as required | as required |

Preferably, when the antibacterial agent is erythromycin or colloidal silver, it will be present in the composition in quantities comprised of between 0.05 and 5% by weight.

When the antibacterial agent is silver sulphadiazine, it will preferably be present in quantities comprised of between 0.01 and 3% by weight.

The composition of the invention is prepared by mixing the desired components with emollient and hydrating substances, for example vaseline and lanolin, until obtaining a creamy consistency. Alternatively, the composition may be formulated as sprays, dermatological powders, gels, ointments, emulsions, drops for external use, solutions, vaginal douches, vaginal ovules, labial sticks; gauzes or plasters impregnated with the composition of the invention may also be envisaged.

The composition of the invention is also employed for veterinary use.

EXAMPLES

Some examples of compositions of the invention are reported below.

Example 1

Table 2. Wound-healing cream

| Ingredients | Quantity (% by weight) |
|---|---|
| Sucralfate | 4.5 |
| glycine | 4.0 |
| Aluminium acetate | 2.0 |
| Vitamin A | 1.0 |
| Anhydrous lanolin | as required |
| White vaseline | as required |

Example 2

Table 3. Wound-healing and antibacterial cream.

| Ingredients | Quantity (% by weight) |
|---|---|
| Sucralfate | 4.0 |
| glycine | 4.0 |
| Aluminium acetate | 2.0 |
| Vitamin A | 1.0 |
| Erythromycin | 3.0 |
| Anhydrous lanolin | as required |
| White vaseline | as required |

Example 3

Table 4. Wound-healing and antifungal cream.

| Ingredients | Quantity (% by weight) |
| --- | --- |
| Sucralfate | 4.0 |
| glycine | 4.0 |
| Aluminium acetate | 2.0 |
| Vitamin A | 1.0 |
| Econazole | 3.0 |
| Anhydrous lanolin | as required |
| White vaseline | as required |

Example 4.

Table 5. Wound-healing and antibacterial cream.

| Ingredients | Quantity (% by weight) |
| --- | --- |
| Sucralfate | 4.0 |
| glycine | 4.0 |
| Aluminium acetate | 2.0 |
| Vitamin A | 1.0 |
| Gentamycin | 0.1 |
| Anhydrous lanolin | as required |
| White vaseline | as required |

Example 5.

Table 6. Wound-healing and antibacterial cream.

| Ingredients | Quantity (% by weight) |
| --- | --- |
| Sucralfate | 4.0 |
| glycine | 4.0 |
| Aluminium acetate | 2.0 |
| Vitamin A | 1.0 |
| Neomycin | 0.5 |
| Anhydrous lanolin | as required |
| White vaseline | as required |

Example 6

Table 7 - Wound-healing, antibacterial and antifungal cream

| Ingredients | Quantity (% by weight) |
|---|---|
| Sucralfate | 4.0 |
| glycine | 4.0 |
| Aluminium acetate | 2.0 |
| Vitamin A | 1.0 |
| Colloidal silver | 0.2 |
| Miconazole | 0.1 |
| Anhydrous lanolin | as required |
| White vaseline | as required |

Example 7

Table 8 - Wound-healing and antibacterial cream.

| Ingredients | Quantity (% by weight) |
|---|---|
| Sucralfate | 4.0 |
| Glycine | 4.0 |
| Aluminium acetate | 2.0 |
| Vitamin A | 1.0 |
| Silver sulphadiazine | 0.1 |
| Anhydrous lanolin | as required |
| White vaseline | as required |

Example 8

Table 9 - Wound-healing and antifungal cream

| Ingredients | Quantity (% by weight) |
|---|---|
| Sucralfate | 4.0 |
| glycine | 4.0 |
| Aluminium acetate | 2.0 |
| Vitamin A | 1.0 |
| itraconazole | 0.5 |
| Anhydrous lanolin | as required |
| White vaseline | as required |

Experimental section.

The composition of example 1 has been tested to assess its capacity to repair wounds induced artificially in vitro in human endothelial cell monolayers. This capacity may be considered an indication of vasorepair activity in vivo. A preliminary cytotoxicity assay (MTT) has been performed in order to assess the sub-toxic concentrations to be used on these cells.

The cellular type used is represented by human primary endothelial cells (HUVEC) which are universally recognised as a model simulating the vasal walls for the *in* vitro study of vasal repair activity and angiogenesis.

When HUVEC cells are exposed to substances capable of stimulating their migratory capacity, an indispensable element in the vasoregeneration and wound repair process, they change shape and alter their cytoskeletal organisation, transforming themselves from cells with a stationary phenotype into cells with a migratory phenotype. The microfilament bundles containing adhesion molecules such as vinculin, actin and stress fibres, keeping the cells anchored to the substrate, disappear or migrate to the cell periphery, allowing cell migration.

Execution of the test

The in vitro experimental model adopted consists of primary cultures of human vascular endothelium, denominated HUVEC. These are vesicular cells derived from the umbilical vein (Source: American Type Culture Collection N° CRL-1730).

Sample preparation

The composition of example 1 is dissolved at a concentration of 1 mg/ml in ethanol and then diluted in cell culture medium at different final concentrations.

MTT cell viability test

Treatment *and exposure*

Cells have been seeded in DMEM + 10% FCS in 24 well plates for 24 hours. Then, fresh culture medium, supplemented with 10% FCS and containing the test product, at four final dilutions comprised of between 1.25 and 0.05 mg/ml, has been added.

The sample has been dissolved directly in culture medium. Each sample has been tested in triplicate and the experiments have been repeated twice. Untreated cells have been used as a negative control, and cells have been treated with a surfactant of known toxicity (sodium lauryl sulphate) dissolved in culture medium at concentrations comprised of between 0.05 mg/ml to 2 $\mu$g/ml as positive controls.

Following the incubation period, the cytotoxicity test (MTT) has then been performed in order to evaluate the percentage cell survival. The MTT test assesses the toxic impact of the substance in question on cell viability.

Description of the test.

The MTT test is simple, accurate and provides reproducible results. This method has originally been developed by Mossman (Mossman, T. (1993), Rapid colorimetric assay for cellular growth and survival: application to proliferation and cytotoxicity assays, J. Immunol. Methods 65:55-63). The key reagent is 3-[4,5-dimethylthiazol-2-yl]-2,5-diphenyl tetrazolium bromide or MTT, a substance which gives a yellow colour in aqueous solution. The mitochondrial dehydrogenases of living cells cleave the tetrazolium ring, leading to the formation of water insoluble purple coloured formazan crystals. The crystals are dissolved in acidified isopropanol and the resulting purple solution is measured spectrophotometrically. Increased or reduced cellular vitality results in concomitant changes in the quantity of formazan formed, which can be considered an indicator of the degree of cytotoxicity caused by exposure to the irritant substances.

Following treatment, the cells are washed with wash solution (Dulbecco's Phosphate Buffered Saline). Following removal of the solution, the medium - MTT is added to each well and the cells are incubated at 37°C.

At the end of the incubation period, the medium-MTT is removed and the MTT solubilising solution is added to each well.

The plates are agitated on a plate shaker ensuring that all the crystals have dissolved and have formed a homogeneous solution. The absorbance is read using a colourimeter fitted with a plate reader, subtracting the blank reading.

Results are expressed as:

$$\% \text{ cellular survival} = \frac{\text{OD of treated cells} \times 100}{\text{OD of untreated cells}}$$

*Expression of the results*

Cytotoxicity data obtained using the MTT test is plotted on a graph against the concentration of the product under test, thus giving a dose-response curve, allowing the determination of:

- the theoretical regression curve;
- the theoretical $IC_{50}$ value (the concentration inhibiting growth by 50%), i.e. the concentration which induces a reduction in cell vitality of 50% with respect to untreated cells and, hence, allows evaluation of the potential irritant nature of the composition.

Cytotoxicity results have been corrected by subtracting the absorbance readings due to the dilution medium.

For the finished products, $LC_{50}$ values < 1 mg/ml may be considered irritants, values > 3 mg/ml show optimal biocompatibility.

Wound healing test

*Treatment and exposure*

Cells have been treated with the composition of the example, at final concentrations of 0.1 and 0.05 mg/ml in culture medium, for 4 days. Cells treated with medium alone or with Vascular Endothelial Growth Factor (VEGF) have been used as negative and positive controls, respectively. The cells are kept in an incubator at 37°C with 5% $CO_2$. Tests are performed in triplicate.

*Preparation of cell cultures*

HUVEC cells, between the 1st and 4th passages, are used. They are plated out at 20,000 cells/well in sterile 24 well plates pre-treated with 10 $\mu$g/ml fibronectin and left to grow to confluency in M199 medium supplemented with 10% FCS, 100 $\mu$g/ml Endothelial Cell Growth Factor and 100 $\mu$g/ml heparin.

*Execution of the wound and exposure of cells to the substance.*

Having reached confluency, an artificial wound is made along the entire length of the monolayer, for each sample, using a sterile plastic tip. The cell culture medium is removed and replaced with medium containing the test substance, except for the negative controls.

*Evaluation of wound repair*

Immediately following execution of the wound (TO) and at various times afterwards, fresh observations are made of the treated samples and the controls by phase contrast microscopy using a Leica DM-IL IMC microscope at 200 and 400X enlargement. The images are documented by digital photographs taken using an Olympus C-100 camera.

Results

*MTT cell viability test*

Indications of cell viability following treatment with various dilutions of the product under test are reported in table 9.

Table 9

| Dose mg/ml | 1.25 | 0.2 | 0.1 | 0.05 |
|---|---|---|---|---|
| % cell viability (with respect to negative controls) | 0 | 61.90 | 95.24 | 95.24 |
| Standard deviation | 0.0 | 2.75 | 0.0 | 2.75 |
| $IC_{50}$ = 0.55 mg/ml | | | | |

*wound healing test*

*Photographic assessment of wound repair*

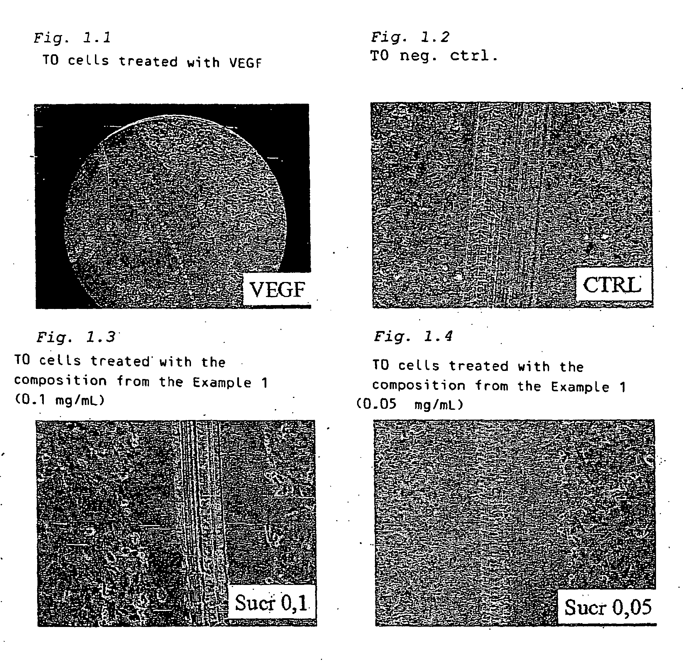

Wound repair activity has been documented photographically over time by comparison with the VEGF-treated and untreated cells, using a phase contrast optical microscope, with digital camera, throughout the execution of the test (see Figures 1.1-4.3).

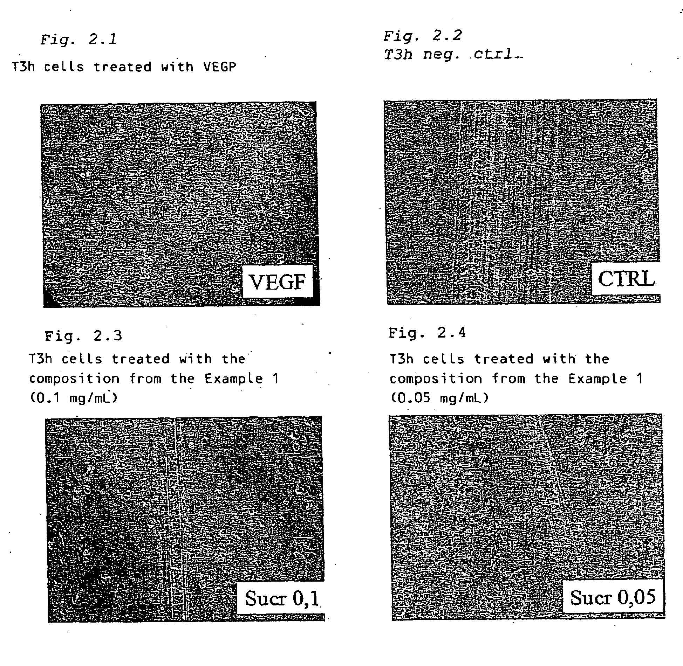

It is clear from the photographic sequence that the product under test induces the conversion of the cellular phenotype from stationary to migratory. The cells treated with the product, just as those treated with VEGF, begin to migrate as early as 3 hours (Fig 2.1, 2.3, 2.4.), while this phenomenon is not observed in the untreated cells (Fig.2.2).

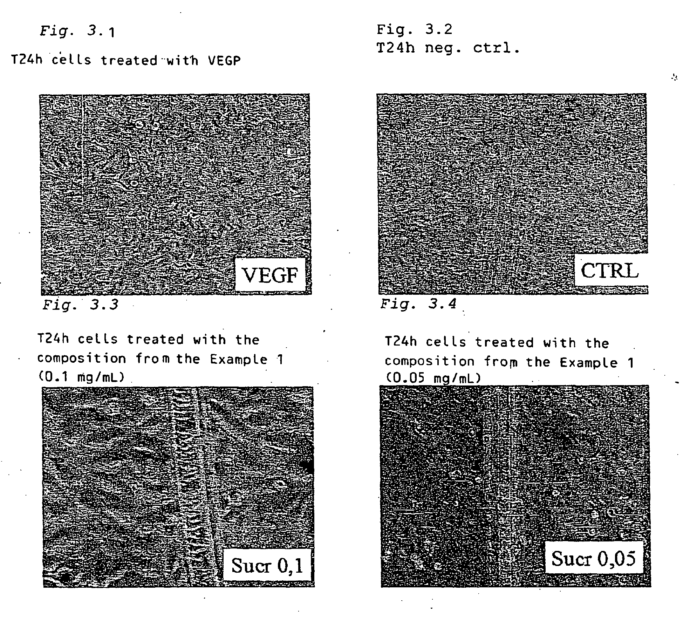

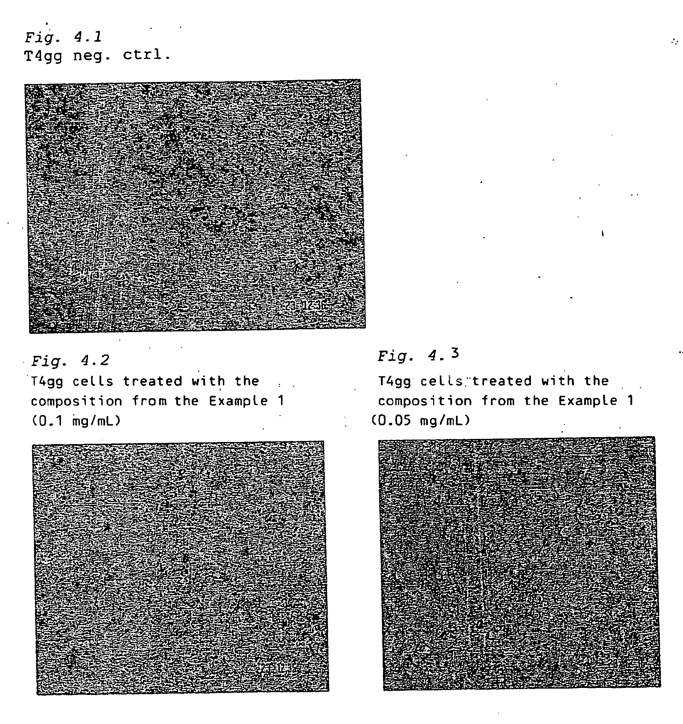

At 24 hours, no differences are observed between the treated and untreated samples, in that in all cases the wound is filled in and the continuous monolayer has reformed (Fig. 3.1, 3.2, 3.3, 3.4). However, by prolonging exposure to 4 days, it is clear that the product under test continues to induce increased cellular proliferation and migration, in a dose-dependent manner, with respect to the untreated cells, so much so, as to induce them to form a bilayer (Fig. 4.1, 4.2, 4.3).

In conclusion, the composition of example 1 shows vascular regeneration stimulatory activity. Indeed, the product stimulates the migration of endothelial cells leading to the repair of wounds and ulcerations of various types.

**Claims**

1.   A composition comprising sucralfate, aluminium acetate, glycine and at least one vitamin selected from vitamin A, C or E.

2.   The composition according to claims 1 wherein said at least one vitamin is vitamin A.

3.   The composition according to claims 1 or 2 comprising an antibacterial agent.

4.   The composition according to claim 3 wherein said antibacterial agent is selected from erythromycin, gentamycin, neomycin, colloidal silver and/or silver sulfadiazine.

5.   The composition according to any of the claims 1 to 4 comprising an antifungal agent.

6.   The composition according to claim 5 wherein said antifungal agent is selected from econazole, miconazole and/or itraconazole.

7.   The composition according to any of the claims 1 to 6 comprising emollient and hydrating agents.

8.   The composition according to claim 7 wherein said emollient and hydrating agents are vaseline and lanolin.

9.   The composition according to any of the claims 1 to 8 wherein said sucralfate is present in quantities ranging from 1% to 6% by weight, preferably from 2 to 5% by weight; said aluminium acetate is present in quantities ranging from 0.5% to 3.5% by weight, preferably from 1% to 2.5% by weight; said at least one vitamin is present in quantities ranging from 0.2% to 3.0% by weight, preferably from 0.8% to 1.5% by weight; said glycine is present in quantities ranging from 1% to 6% by weight, preferably from 2% to 5% by weight.

10.   The composition according to any of the claims 3 to 9 wherein said antibacterial agent is present in quantities ranging from 0.01% to 5% by weight, preferably from 2% to 4% by weight and said antifungal agent is present in quantities ranging from 0.01% to 5% by weight, preferably from 0.1% to 4% by weight.

**11.** A pharmaceutical composition according to any of the claims 1 to 10, said composition being selected from: a) 4.5% sucralfate, 4.0% glycine, 2.0% aluminium acetate, 1.0% vitamin A, lanolin and vaseline; b) 4.0% sucralfate, 4.0% glycine, 2.0% aluminium acetate, 1.0% vitamin A, 3.0% erythromycin, lanolin and vaseline; c) 4.0% sucralfate, 4.0% glycine, 2.0% aluminium acetate, 1.0% vitamin A, 3.0% econazole, lanolin and vaseline; d) 4.0% sucralfate, 4.0% glycine, 2.0% aluminium acetate, 1.0% vitamin A, 0.1% gentamycin, lanolin and vaseline; e)4.0% sucralfate, 4.0% glycine, 2.0% aluminium acetate, 1.0% vitamin A, 0.5% neomycin, lanolin and vaseline; f) 4.0% sucralfate, 2.0% aluminium acetate, 1.0% vitamin A, 0.2% colloidal silver, 0.1% miconazole, lanolin and vaseline, g) 4.0% sucralfate, 2.0% aluminium acetate, 1.0% vitamin A, 0.1% silver sulfadiazine, lanolin and vaseline; h) 4.0% sucralfate, 2.0% aluminium acetate, 1.0% vitamin A, 0.5% itraconazole, lanolin and vaseline.

**12.** The composition according to any of the claims 1 to 11, wherein said composition is formulated in a form selected from sprays, dermatological powders, gels, ointments, emulsions, drops for external use, solutions, vaginal douches, vaginal ovules, labial sticks, gauzes or plasters.

**13.** The composition according to any of the claims 1 to 11 for use as a medicament.

**14.** Use of the composition according to any of the claims 1 to 11 for the preparation of a medicament for the treatment of vaginal and epidermal wounds.

**15.** The use according to claim 14 for the treatment and protection of epithelial and vaginal ulcerations.

**16.** The use of the composition according to any of the claims 3 to 11 for the preparation of a medicament for the treatment of bacterially and/or fungally infected wounds.

**Patentansprüche**

**1.** Eine Zusammensetzung umfassend Sucralfat, Aluminiumacetat, Glycin und wenigstens ein Vitamin ausgewählt aus Vitamin A, C oder E.

**2.** Die Zusammensetzung nach Anspruch 1, worin wenigstens ein Vitamin Vitamin A ist.

**3.** Die Zusammensetzung nach Anspruch 1 oder 2 umfassend ein antibakterielles Mittel.

**4.** Die Zusammensetzung nach Anspruch 3, worin das genannte antibakterielle Mittel ausgewählt ist aus Erythromycin, Gentamycin, Neomycin, kolloidalem Silber und/oder Silbersulfadiazin.

**5.** Die Zusammensetzung nach irgendeinem der Ansprüche 1 bis 4 umfassend ein fungizides Mittel.

**6.** Die Zusammensetzung nach Anspruch 5, worin das genannte fungizide Mittel ausgewählt ist aus Econazol, Miconazol und/oder Itraconazol.

**7.** Die Zusammensetzung nach irgendeinem der Ansprüche 1 bis 6 umfassend Weichmacher und Feuchthaltemittel.

**8.** Die Zusammensetzung nach Anspruch 7, worin die genannten Weichmacher und Feuchthaltemittel Vaseline und Lanolin sind.

**9.** Die Zusammensetzung nach irgendeinem der Ansprüche 1 bis 8, worin das genannte Sucralfat in Mengen im Bereich von 1 Gewichts-% bis 6 Gewichts-%, bevorzugt von 2 bis 5 Gewichts-% vorliegt; das genannte Aluminiumacetat in Mengen im Bereich von 0,5 Gewichts-% bis 3,5 Gewichts-%, bevorzugt von 1 Gewichts-% bis 2,5 Gewichts-% vorliegt; das genannte wenigstens eine Vitamin in Mengen von 0,2 Gewichts-% bis 3,0 Gewichts-%, bevorzugt von 0,8 Gewichts-% bis 1,5 Gewichts-% vorliegt; das genannte Glycin in Mengen im Bereich von 1 Gewichts-% bis 6 Gewichts-%, bevorzugt von 2 Gewichts-% bis 5 Gewichts-% vorliegt.

**10.** Die Zusammensetzung nach irgendeinem der Ansprüche 3 bis 9, worin das genannte antibakterielle Mittel in Mengen im Bereich vom 0,01 Gewichts-% bis 5 Gewichts-%, bevorzugt von 2 Gewichts-% bis 4 Gewichts-% vorliegt und das genannte fungizide Mittel in Mengen von 0,01 Gewichts-% bis 5 Gewichts-%, bevorzugt von 0,1 Gewichts-% bis 4 Gewichts-% vorliegt.

**11.** Eine pharmazeutische Zusammensetzung nach irgendeinem der Ansprüche 1 bis 10, wobei die genannte Zusammensetzung ausgewählt ist aus: a) 4,5 % Sucralfat, 4,0 % Glycin, 2,0 % Aluminiumacetat, 1,0 % Vitamin A, Lanolin und Vaseline; b) 4,0 % Sucralfat, 4,0 % Glycin, 2,0 % Aluminiumacetat, 1,0 % Vitamin A, 3,0 % Erythromycin, Lanolin und Vaseline; c) 4,0 % Sucralfat, 4,0 % Glycin, 2,0 % Aluminiumacetat, 1,0 % Vitamin A, 3,0% Econazol, Lanolin und Vaseline; d) 4,0% Sucralfat, 4,0% Glycin, 2,0 % Aluminiumacetat, 1,0 % Vitamin A, 0,1 % Gentamycin, Lanolin und Vaseline; e) 4,0 % Sucralfat, 4,0 % Glycin, 2,0 Aluminiumacetat, 1,0 % Vitamin A, 0,5 % Neomycin, Lanolin und Vaseline; f) 4,0 % Sucralfat, 2,0 % Aluminiumacetat, 1,0 %Vitamin A, 0,2 % kolloidales Silber, 0,1 % Miconazol, Lanolin und Vaseline; g) 4,0 % Sucralfat, 2,0 % Aluminiumacetat, 1,0 % Vitamin A, 0,1 % Silbersulfadiazin, Lanolin und Vaseline; h) 4,0% Sucralfat, 2,0 % Aluminiumacetat, 1,0 % Vitamin A, 0,5% Itraconazol, Lanolin und Vaseline.

**12.** Die Zusammensetzung nach irgendeinem der Ansprüche 1 bis 11, worin die genannte Zusammensetzung in einer Form ausgewählt aus Sprays, dermatologischen Pudern, Gelen, Salben, Emulsionen, Tropfen für äußerliche Anwendung, Lösungen, Vaginalspülungen, Vaginal-Ovulen, Lippenstiften, Gazen oder Pflastern formuliert ist.

**13.** Die Zusammensetzung nach irgendeinem der Ansprüche 1 bis 11 zur Verwendung als Medikament.

**14.** Verwendung der Zusammensetzung nach irgendeinem der Ansprüche 1 bis 11 zur Herstellung eines Medikaments zur Behandlung von vaginalen und epidermalen Wunden.

**15.** Die Verwendung nach Anspruch 14 zur Behandlung und zum Schutz vor Epithel- und Vaginalgeschwüren.

**16.** Die Verwendung der Zusammensetzung nach irgendeinem der Ansprüche 3 bis 11 zur Herstellung eines Medikaments für die Behandlung bakteriell und / oder durch Pilze infizierter Wunden.

**Revendications**

**1.** Composition comprenant du sucralfate, de l'acétate d'aluminium, de la glycine et au moins une vitamine choisie parmi la vitamine A, C ou E.

**2.** Composition selon la revendication 1, dans laquelle ladite au moins une vitamine est la vitamine A.

**3.** Composition selon la revendication 1 ou 2, comprenant un agent antibactérien.

**4.** Composition selon la revendication 3, dans laquelle ledit agent antibactérien est choisi parmi l'érythromycine, la gentamycine, la néomycine, l'argent colloïdal et/ou la sulfadiazine d'argent.

**5.** Composition selon l'une quelconque des revendications 1 à 4, comprenant un agent antifongique.

**6.** Composition selon la revendication 5, dans laquelle ledit agent antifongique est choisi parmi l'éconazole, le miconazole et/ou l'itraconazole.

**7.** Composition selon l'une quelconque des revendications 1 à 6, comprenant des agents émollients et hydratants.

**8.** Composition selon la revendication 7, dans laquelle lesdits agents émollients et hydratants sont la vaseline et la lanoline,

**9.** Composition selon l'une quelconque des revendications 1 à 8, dans laquelle ledit sucralfate est présent en des quantités comprises dans la plage allant de 1 % à 6 % en poids, de préférence de 2% à 5 % en poids ; ledit acétate d'aluminium est présent en des quantités comprises dans la plage allant de 0,5 % à 3,5 % en poids, de préférence de 1 % à 2,5 % en poids ; ladite au moins une vitamine est présente en des quantités comprises dans la plage allant de 0,2% à 3,0 % en poids, de préférence de 0,8 % à 1,5 % en poids ; ladite glycine est présente en des quantités comprises dans la plage allant de 1 % à 6 % en poids, de préférence de 2 % à 5 % en poids.

**10.** Composition selon l'une quelconque des revendications 3 à 9, dans laquelle l'agent antibactérien est présent en des quantités comprises dans la plage allant de 0,01 % à 5 % en poids, de préférence de 2 % à 4 % en poids et ledit agent antifongique est présent en des quantités comprises dans la plage allant de 0,01 % à 5 % en poids, de préférence de 0,1 % à 4 % en poids.

11. Composition selon l'une quelconque des revendications 1 à 10, ladite composition étant choisie parmi : a) 4,5 % de sucralfate, 4,0 % de glycine, 2,0 % d'acétate d'aluminium, 1,0 % de vitamine A, de la lanoline et de la vaseline ; b) 4,0 % de sucralfate, 4,0 % de glycine, 2,0 % d'acétate d'aluminium, 1,0 % de vitamine A, 3,0 % d'érythromycine, de la lanoline et de la vaseline ; c) 4,0 % de sucralfate, 4,0 % de glycine, 2,0 % d'acétate d'aluminium, 1,0 % de vitamine A, 3,0 % d'éconazole, de la lanoline et de la vaseline ; d) 4,0 % de sucralfate, 4,0 % de glycine, 2,0 % d'acétate d'aluminium, 1,0 % de vitamine A, 0,1 % de gentamycine, de la lanoline et de la vaseline ; e) 4,0 % de sucralfate, 4,0 % de glycine, 2,0 % d'acétate d'aluminium, 1,0 % de vitamine A, 0,5 % de néomycine, de la lanoline et de la vaseline ; f) 4,0 % de sucralfate, 2,0 % d'acétate d'aluminium, 1,0 % de vitamine A, 0,2 % d'argent colloïdal, 0,1 % de miconazole, de la lanoline et de la vaseline ; g) 4,0 % de sucralfate, 2,0 % d'acétate d'aluminium, 1,0 % de vitamine A, 0,1 % de sulfadiazine d'argent, de la lanoline et de la vaseline; h) 4,0 % de sucralfate, 2,0 % d'acétate d'aluminium, 1,0 % de vitamine A, 0,5 % d'itracanazole, de la lanoline et de la vaseline.

12. Composition selon l'une quelconque des revendications 1 à 11, dans laquelle ladite composition est formulée sous une forme choisie parmi les sprays, les poudres dermatologiques, les gels, les pommades, les émulsions, les gouttes pour une utilisation externe, les solutions, les douches vaginales, les ovules vaginaux, les sticks labiaux, les gazes ou les pansements,

13. Composition selon l'une quelconque des revendications 1 à 11, pour une utilisation en tant que médicament.

14. Composition selon l'une quelconque des revendications 1 à 11, pour la préparation d'un médicament pour le traitement de plaies vaginales et épidermiques.

15. Utilisation selon la revendication 14, pour le traitement et la protection d'ulcérations épithéliales et vaginales.

16. Composition selon l'une quelconque des revendications à 11, pour la préparation d'un médicament pour le traitement de plaies infectées par des bactéries et/ou des champignons.

Fig. 1.1
TO cells treated with VEGF

Fig. 1.2
TO neg. ctrl.

Fig. 1.3
TO cells treated with the
composition from the Example 1
(0.1 mg/mL)

Fig. 1.4
TO cells treated with the
composition from the Example 1
(0.05 mg/mL)

Fig. 2.1

T3h cells treated with VEGP

Fig. 2.2
T3h neg. ctrl.

VEGF

CTRL

Fig. 2.3

T3h cells treated with the
composition from the Example 1
(0.1 mg/mL)

Fig. 2.4

T3h cells treated with the
composition from the Example 1
(0.05 mg/mL)

Sucr 0,1

Sucr 0,05

Fig. 3.1

T24h cells treated with VEGP

Fig. 3.2
T24h neg. ctrl.

VEGF

CTRL

Fig. 3.3

T24h cells treated with the
composition from the Example 1
(0.1 mg/mL)

Fig. 3.4

T24h cells treated with the
composition from the Example 1
(0.05 mg/mL)

Sucr 0,1

Sucr 0,05

*Fig. 4.1*
T4gg neg. ctrl.

*Fig. 4.2*
T4gg cells treated with the
composition from the Example 1
(0.1 mg/mL)

*Fig. 4.3*
T4gg cells treated with the
composition from the Example 1
(0.05 mg/mL)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 8905645 A

**Non-patent literature cited in the description**

- **MOSSMAN, T.** Rapid colorimetric assay for cellular growth and survival: application to proliferation and cytotoxicity assays. *J. Immunol. Methods,* 1993, vol. 65, 55-63